# EUROPÄISCHE PATENTSCHRIFT

(11) **EP 3 768 412 B1**
(45) Veröffentlichungstag und Bekanntmachung des Hinweises auf die Patenterteilung: **17.07.2024**
(21) Anmeldenummer: 19712171.8
(22) Anmeldetag: 18.03.2019
(51) Int. Cl.: B01D 61/18, B01D 63/06, B01D 65/00, A61L 9/16, B01D 46/00, B01D 46/24, B01D 46/52, B01D 71/36, B01D 71/68

(54) **FILTERMODUL MIT RANDVERSTÄRKTER MEMBRAN, VERFAHREN ZUR HERSTELLUNG DES FILTERMODULS SOWIE DESSEN VERWENDUNG**
FILTER MODULE HAVING AN EDGE-REINFORCED MEMBRANE, METHOD FOR PRODUCING THE FILTER MODULE AND USE THEREOF
MODULE DE FILTRATION À MEMBRANE AVEC BORD RENFORCÉ, PROCÉDÉ DE FABRICATION DU MODULE DE FILTRATION ET UTILISATION DUDIT MODULE DE FILTRATION

(30) Priorität: 19.03.2018 DE 102018002261
(43) Veröffentlichungstag der Anmeldung: 27.01.2021
(73) Patentinhaber: Sartorius Stedim Biotech GmbH, 37079 Göttingen (DE)
(72) Erfinder: NIKOLOUDIS, Paschalis, 37079 Göttingen (DE); HANDT, Sebastian, 37085 Göttingen (DE); LOEWE, Thomas, 37077 Göttingen (DE)
(74) Vertreter: Novagraaf International SA
(86) Internationale Anmeldenummer: PCT/EP2019/056696
(87) Internationale Veröffentlichungsnummer: WO 2019/179938

(56) Entgegenhaltungen:
- EP-A2- 0 139 822
- DE-U1- 9 305 041
- US-B1- 6 511 600

## Beschreibung

Die vorliegende Erfindung betrifft ein Filtermodul mit randverstärkter Membran, ein Verfahren zur Herstellung des Filtermoduls sowie dessen Verwendung.

Im Stand der Technik sind Filtermodule für den Einsatz in der Steril- und Virenfiltration beschrieben, welche über Verankerungselemente verfügen, in denen die beiden Enden einer röhrenförmigen, gegebenenfalls plissierten Filtermembran eingebettet sind. Die Einbettung erfolgt üblicherweise durch Eintauchen der Randbereiche der Membran in die erweichten Verankerungselemente, welche auch als Endkappen bezeichnet werden. Ein solches Abdichten der Membran mit den Endkappen soll die Sterilität des Filtermoduls gewährleisten.

Problematisch ist hierbei allerdings, dass bei der vorstehend erwähnten Einbettung die Verbindung der Filtermembran mit dem Verankerungselement häufig unzureichend ist. Ein Grund hierfür liegt in den unterschiedlichen Materialien, aus denen die Membran und die für die Abdichtung verwendeten Endkappen aufgebaut sind. Während beispielsweise im Fall von Luftfiltern die Filtermembranen häufig aus Polytetrafluorethylen bestehen, welches sich durch superabweisende Eigenschaften auszeichnet, sind die Endkappen häufig aus Polyolefinen wie etwa Polypropylen gebildet. Die unterschiedlichen Oberflächenenergien führen naturgemäß zu einer schlechten Verbindung der eingesetzten Materialien. Diese Schwierigkeit bei der Herstellung einer sterilen Verbindung ist auch in DE9305041 U1 behandelt, wo eine Umspritzung des Rahmenelementes vorgeschlagen wird. In der Regel werden bei der Einbettung lediglich die Endkappen, jedoch nicht die Filtermembranen angeschmolzen. Auch dieser Umstand wirkt sich nachteilig auf die Verbindung der Membran mit dem Verankerungselement aus.

Sind die Membran und das Verankerungselement nicht ausreichend miteinander verbunden, kann dies zum Auftreten von Undichtigkeiten im so erhaltenen Filtermodul führen, was wiederum eine mangelnde Sterilität zur Folge hat. Gerade in der Steril- und Virenfiltration ist jedoch eine nahezu vollständige Bakterien- und Virenfreiheit unerlässlich.

Vor diesem Hintergrund liegt der vorliegenden Erfindung die Aufgabe zugrunde, ein Filtermodul bereitzustellen, in welchem die Membran und das zur Abdichtung verwendete Verankerungselement derart miteinander verbunden sind, dass eine ausreichende Sterilität des Filtermoduls auch über einen längeren Benutzungszeitraum gewährleistet ist. Weiterhin ist es eine Aufgabe der vorliegenden Erfindung, ein Verfahren zur Herstellung eines solchen Filtermoduls bereitzustellen.

Die vorstehend definierten Aufgaben werden durch die in den Ansprüchen gekennzeichneten Ausführungsformen der vorliegenden Erfindung gelöst.

In einem ersten Aspekt betrifft die vorliegende Erfindung ein Filtermodul, welches eine Membran mit einem darauf angeordneten porösen Randgebilde umfasst und weiter mindestens ein Verankerungselement umfasst, wobei die Membran mit dem darauf angeordneten porösen Randgebilde in das mindestens eine Verankerungselement eingebettet ist.

Durch das auf der Membran angeordnete poröse Randgebilde, welches als Randverstärkung bei der Einbettung der Membran in das mindestens eine Verankerungselement dient, d.h. zusammen mit der Membran in das Verankerungselement eingebettet ist, wird die Verbindung der vorstehenden Komponenten im so erhaltenen Filtermodul verbessert. Insbesondere kann hierdurch das Auftreten von Undichtigkeiten im Bereich der Einbettung nahezu vollständig unterbunden werden, vor allem wenn die Membran und das darauf angeordnete poröse Randgebilde als Verbund vorliegen. Das erfindungsgemäße Filtermodul mit randverstärkter Membran ist im Randbereich fluiddicht und weist somit eine ausgezeichnete Sterilität auch über einen längeren Benutzungszeitraum auf, wodurch es für eine Vielzahl von Anwendungen in Frage kommt. In besonderer Weise eignet sich das erfindungsgemäße Filtermodul zur Steril- und Virenfiltration von fluiden Medien, insbesondere von gasförmigen Medien, vor allem von Luft, wie auch von flüssigen Medien.

Überraschenderweise wurde im Rahmen der vorliegenden Erfindung festgestellt, dass aufgrund der Porosität sowie der Strukturierung bzw. Oberflächenrauheit der Randverstärkung ein Verklammerungseffekt zwischen der Filtermembran und dem mindestens einen Verankerungselement entsteht, wobei sich die Randverstärkung gewissermaßen sowohl in die Filtermembran als auch in das mindestens eine Verankerungselement verkrallt. Die Strukturierung bzw. Oberflächenrauheit der Randverstärkung wird zunächst durch die von den Poren im porösen Randgebilde, welches eine Porengröße von mindestens 0,1 µm aufweist, aufgespannten Freiräume bedingt und kann schließlich während der Herstellung des erfindungsgemäßen Filtermoduls gezielt gesteuert werden, wie weiter unten ausführlicher beschrieben.

Im erfindungsgemäßen Filtermodul unterliegt die Filtermembran, nachstehend der Einfachheit halber nur als Membran bezeichnet, keiner besonderen Einschränkung.

Entsprechend des Verwendungszwecks, d.h. je nach Größe der abzutrennenden Teilchen, weist die Membran eine geeignete Porengröße auf, welche typischerweise im Bereich von 0,005 bis 10 µm liegt, ohne jedoch hierauf beschränkt zu sein. Bevorzugt ist für die Porengröße der Membran ein Bereich von 0,01 bis 1,2 µm, wobei ein Bereich von 0,02 bis 0,45 µm besonders bevorzugt ist.

Zur Bestimmung der Porengröße wird erfindungsgemäß bei Porengrößen von mindestens 0,1 µm, d.h. für Mikrofiltrationsmembranen mit einer mittleren Porengröße von 0,1 bis 10 µm, die Kapillarfluss-Porometrie verwendet. Hierbei handelt es sich um eine Gas/Flüssigkeits-Porosimetrie, bei der die differentiellen Gasdrücke und Flussraten durch eine Membranprobe zuerst im feuchten und anschließend im trockenen Zustand gemessen werden. Die Membranprobe wird vor der Messung mit einer benetzenden Flüssigkeit so in Kontakt gebracht, dass sämtliche vorhandenen Poren mit dieser Flüssigkeit gefüllt sind. Nach dem Füllen der Poren und dem Einbringen der Probe ist die Messzelle zu verschließen und die Messung zu starten. Der Gasdruck wird nach dem Start der Messung automatisch und schrittweise erhöht und die dem anliegenden Druck entsprechenden Porendurchmesser werden durch den Gasdruck entleert. Dies erfolgt solange, bis der relevante Porenbereich erfasst wurde, d.h. bis auch die kleinsten im Messbereich vorhandenen Poren von der Flüssigkeit befreit sind. Danach wird der Druck wieder abgesenkt und die Messung an der nun trockenen Probe automatisch wiederholt. Aus der Differenz beider Druck-Flussrate-Kurven wird die Porengrößenverteilung über die Young-Laplace-Gleichung berechnet (s. auch A. Shrestha, "Characterization of porous membranes via porometry", 2012, Mechanical Engineering Graduate Theses & Dissertations, Paper 38, University of Colorado at Boulder).

Bei Porengrößen von höchstens 0,1 µm wird erfindungsgemäß die Flüssig-Flüssig-Verdrängungsmethode angewendet. Diese weist Ähnlichkeiten zur Kapillarfluss-Porometrie auf. Allerdings werden hier nicht die Gasflussraten, sondern die Flussraten der verdrängenden Flüssigkeit in Abhängigkeit der differentiellen Druckerhöhung gemessen (s. auch R. Dävila, "Characterization of ultra and nanofiltration commercial filters by liquid-liquid displacement porosimetry", 2013).

Was die Dicke der Membran anbelangt, ist die vorliegende Erfindung ebenfalls nicht weiter eingeschränkt. Beispielsweise kann die Membrandicke von 20 bis 400 µm betragen, wobei ein Bereich von 20 bis 300 µm bevorzugt ist.

Die geometrische Form der Membran unterliegt erfindungsgemäß ebenfalls keiner besonderen Einschränkung, solange die Membran einen Randbereich aufweist, welcher mit dem porösen Randgebilde versehen werden kann und welcher zum Eintauchen in das mindestens eine Verankerungselement geeignet ist. In einer Ausführungsform weist die Membran eine rechteckige Form auf, wobei beispielsweise einer der vier Randbereiche oder zwei gegenüberliegende Randbereiche der Membran mit dem porösen Randgebilde versehen ist/sind.

In einer bevorzugten Ausführungsform ist die Membran mit dem darauf angeordneten porösen Randgebilde röhrenförmig. Hierzu kann eine rechteckige Membran als Grundlage dienen, welche dann zu einer Röhre geschlossen wird, beispielsweise durch Verschweißen. Durch den Röhrenschluss wird schließlich eine zylinderförmige Anordnung mit offenen Stirnflächen erhalten. Der Röhrenschluss erfolgt dabei derart, dass das auf der Membran angeordnete poröse Randgebilde einen der beiden Randbereiche bzw. beide Randbereiche auf der Röhrenaußenseite entlang des Röhrenumfangs bedeckt. Auf analoge Weise kann ausgehend von einer rechteckigen Membran auch eine prismenförmige Membran erhalten werden, welche anstelle eines kreisförmigen Querschnitts einen vieleckigen, beispielsweise einen quadratischen Querschnitt aufweist. Allgemein werden Filtermodule mit röhrenförmiger und gegebenenfalls plissierter Membran als Filterkerzen bezeichnet.

In einer bevorzugten Ausführungsform der vorliegenden Erfindung ist die Membran aus einem Polymer, ausgewählt aus der Gruppe, bestehend aus Polytetrafluorethylen (PTFE), Polyvinylidenfluorid (PVDF), Polyethersulfon (PES), Polyamid, Polyolefin, Celluloseacetat (CA) und Cellulose, oder aus Gemischen hiervon aufgebaut.

Vorzugsweise ist die Membran des erfindungsgemäßen Filtermoduls aus PTFE aufgebaut. Polytetrafluorethylen weist eine besonders hohe chemische Beständigkeit über die gesamte pH-Wert-Skala auf. Ferner sind Membranen aus PTFE, wie bereits vorstehend erwähnt, superabweisend, d.h. sie zeigen sowohl hydrophobe als auch oleophobe Eigenschaften, weswegen sie für die Filtration von nahezu allen gängigen Lösungsmitteln geeignet sind. Darüber hinaus zeichnen sich Membranen aus Polytetrafluorethylen durch ihre thermische Beständigkeit aus, wodurch sie auch bei erhöhter Temperatur, beispielsweise bei bis zu 260 °C dauerhaft eingesetzt werden können.

Ist die Membran aus Polytetrafluorethylen aufgebaut, so ist es weiterhin bevorzugt, dass es sich hierbei um expandiertes Polytetrafluorethylen (ePTFE) handelt. Durch Expandieren des Polytetrafluorethylens während des Verarbeitungsprozesses werden unter Abnahme der Dichte die einzelnen PTFE-Stränge orientiert.

In einer anderen bevorzugten Ausführungsform ist die Membran des erfindungsgemäßen Filtermoduls aus PES aufgebaut. Polyethersulfone zeichnen sich durch ihre hohe Beständigkeit gegenüber Chemikalien, durch ihre hohe thermische Stabilität sowie durch ihre hohe Schlagzähigkeit aus. Durch Zusatz von Polyvinylpyrrolidon (PVP) bzw. durch eine Oberflächenmodifizierung können dem PES hydrophile Eigenschaften verliehen werden. Gemäß der vorliegenden Erfindung ist die Oberflächenmodifizierung allerdings nicht hierauf beschränkt. So kann die Oberfläche einer aus PES aufgebauten Membran auch anders modifiziert sein, wie beispielsweise in DE 10 2010 044 648 B4 beschrieben.

Erfindungsgemäß ist auf der Membran des Filtermoduls ein poröses Randgebilde angeordnet, welches einen Randbereich der Membran bedeckt. Gemäß der vorliegenden Erfindung ist die Membran also nicht vollständig bedeckt. Wenn vorliegend von dem auf der Membran angeordneten porösen Randgebilde die Rede ist, so meint dies, dass das poröse Randgebilde auf einem Randbereich der Membran angeordnet ist.

Wie bereits vorstehend ausgeführt, ist die Anordnung des porösen Randgebildes auf der Membran nicht auf einen einzigen Randbereich der Membran beschränkt. Insbesondere kann, falls die Membran rechteckig bzw. röhren- oder prismenförmig ist, wie vorstehend beschrieben, das poröse Randgebilde auf einem Randbereich oder auf zwei gegenüberliegenden Randbereichen der Membran angeordnet sein. Solange das poröse Randgebilde einen Randbereich der Membran zu bedecken vermag, unterliegt seine geometrische Form keiner besonderen Einschränkung. Beispielsweise weist das poröse Randgebilde eine Streifenform auf, d.h. es ist rechteckig, wobei der Streifen des porösen Randgebildes einen Randbereich der Membran bedeckt. Gemäß der vorliegenden Erfindung kann es sich bei dem porösen Randgebilde beispielsweise um einen porösen Film oder insbesondere um eine Membran handeln, die gemäß einer bevorzugten Ausführungsform in Streifenform, beispielsweise durch vorhergehendes Schneiden eines entsprechenden flächigen Materials, erhalten werden können.

Liegt das poröse Randgebilde beispielsweise in Form eines Streifens vor, so beträgt dessen Breite typischerweise von 2 bis 10 mm, wobei der durch einen einzelnen Randstreifen bedeckte Bereich etwa 0,5 bis 25% der Gesamtfläche der Filtermembran ausmachen kann, ohne jedoch hierauf beschränkt zu sein.

Durch Verbinden der Membran und des auf ihr angeordneten porösen Randgebildes mittels Wärme und Druck, im Folgenden gelegentlich auch als Siegeln oder Heißsiegeln bezeichnet, kann ein fester Verbund erhalten werden, wobei der Fachmann routinemäßig die erforderlichen Prozessbedingungen für den Verbindungsvorgang auswählt. Je nach Zusammenhang ist im Folgenden unter dem auf der Membran angeordneten porösen Randgebilde entweder die Anordnung vor oder nach dem Verbinden zu verstehen. In einer bevorzugten Ausführungsform der vorliegenden Erfindung liegt die Membran mit dem darauf angeordneten porösen Randgebilde als Verbund vor, wodurch das erfindungsgemäße Filtermodul im Randbereich an der Position des mindestens einen Verankerungselements besonders fluiddicht ist.

Was die Porosität des porösen Randgebildes anbelangt, ist es gemäß Anspruch 1 gefordert, dass das poröse Randgebilde eine Porengröße von mindestens 0,1 µm, besonders bevorzugt von mindestens 0,2 µm, mehr bevorzugt von mindestens 0,4 µm und noch mehr bevorzugt von mindestens 0,5 µm aufweist, beispielsweise mindestens 0,6 µm. Nach oben hin ist die Porengröße des porösen Randgebildes nicht besonders eingeschränkt, wobei sie vorzugsweise höchstens 10 µm beträgt. Dementsprechend kann das poröse Randgebilde eine Porengröße aufweisen, welche die Porengröße der Membran übersteigt.

Sind die Poren des porösen Randgebildes zu klein, so bleibt die Verkrallung mit der erweichten Endkappe aus oder sie ist unzureichend. In gleicher Weise gilt dies auch für die Verkrallung des porösen Randgebildes mit der Membran. Der Verklammerungseffekt zwischen der Filtermembran und dem mindestens einen Verankerungselement, hervorgerufen durch die Randverstärkung, ist in diesem Fall dann lediglich schwach ausgeprägt.

Die Porengröße des porösen Randgebildes wird ebenfalls mit Hilfe der Kapillarfluss-Porometrie bestimmt, wie vorstehend im Zusammenhang mit der Filtermembran beschrieben.

Generell lässt sich eine bestimmte Porengröße beispielsweise während des Herstellungsprozesses der Membran durch Verwendung eines inerten Porenbildners (Porogens) geeigneter Teilchengröße einstellen. Auch kann die Porengröße beispielsweise über mechanisches Recken eines entsprechenden Films eingestellt werden.

Aufgrund der spezifischen Porositäten kann beim Eintauchen der Membran mit dem darauf angeordneten porösen Randgebilde in das erweichte Verankerungselement eine besonders ausgeprägte Verbindung der vorstehenden Komponenten erreicht werden. So werden die Poren des porösen Randgebildes durch das angeschmolzene Material der Endkappe durchdrungen. Zu diesem Zweck ist es weiter bevorzugt, dass das poröse Randgebilde eine bestimmte Strukturierung bzw. Oberflächenrauheit aufweist, wodurch die Verbindung der randverstärkten Membran mit dem Verankerungselement weiter verbessert wird.

Die Strukturierung bzw. Oberflächenrauheit lässt sich während des Verbindens des porösen Randgebildes mit der Filtermembran erzeugen, da hierbei eine Struktur gewissermaßen mit eingepresst wird. Dies geschieht beispielsweise durch das Transportieren der Filtermembran mit dem darauf angeordneten porösen Randgebilde durch ein Heißsiegelungsgerät mittels eines strukturierten Förderbandes oder durch das Mitführen eines Vlieses. In beiden Fällen wird die jeweilige Faserstruktur des umgebenden Mediums in das poröse Randgebilde geprägt, wodurch sogenannte Faserabdrücke erhalten werden. Die Strukturierung bzw. Oberflächenrauheit kann mit Hilfe der Rasterelektronenmikroskopie (REM) bestimmt werden und entspricht näherungsweise der Eindringtiefe der Faser bzw. der durch den Faserdurchmesser bestimmten Spaltbreite, wie weiter unten beschrieben.

Gemäß der vorliegenden Erfindung ist es bevorzugt, dass das auf der Membran angeordnete poröse Randgebilde bündig mit dem Membranende abschließt, d.h. die Filtermembran und die Randverstärkung sind in diesem Fall bündig geschnitten. Keine der beiden vorstehenden Komponenten ragt in diesem Fall über die andere hinaus, wodurch es bei der Einbettung in das Verankerungselement zu einer besonders ausgeprägten Verbindung mit der Endkappe kommt. Insbesondere wird der Randbereich der Membran beim Eintauchen in das erweichte Verankerungselement stabilisiert, da das bündige Abschließen von Membran und darauf angeordnetem porösen Randgebilde die Gefahr eines Ab- bzw. Umknickens des Membranrandbereichs, welcher je nach Materialbeschaffenheit eine hohe Elastizität aufweist, verringert.

Die vorliegende Erfindung ist jedoch nicht auf ein bündiges Abschließen des auf der Membran angeordneten porösen Randgebildes mit dem Membranende beschränkt. So kann das Membranende auch über die Randverstärkung hinausragen, d.h. das poröse Randgebilde kann einen geringfügigen Abstand zum Membranende aufweisen, beispielsweise von bis zu 1 mm, bevorzugt 0,5 mm und - im Einklang mit den vorstehenden Ausführungen - besonders bevorzugt 0 mm.

Die Dicke des porösen Randgebildes unterliegt erfindungsgemäß keiner besonderen Einschränkung. Sie kann beispielsweise 1/5 bis 5/1 der Dicke der Membran betragen, d.h. die Dicke des porösen Randgebildes kann grundsätzlich kleiner oder größer als die Dicke der Membran sein. Typische Dicken des porösen Randgebildes liegen im Bereich von 50 bis 400 µm, bevorzugt im Bereich von 70 bis 300 µm, ohne jedoch hierauf beschränkt zu sein.

Wie bereits vorstehend erwähnt, weist das poröse Randgebilde bevorzugt eine Strukturierung bzw. Oberflächenrauheit auf, welche sich gezielt während der Herstellung steuern lässt und sich über die mittlere Eindringtiefe des Faserabdrucks sowie über die mittlere Spaltbreite, welche in erster Näherung dem mittleren Faserdurchmesser entspricht, definieren lässt. Die mittlere Eindringtiefe des Faserabdrucks beträgt bevorzugt von 1 bis 200 µm, besonders bevorzugt von 1,5 bis 100 µm. Die mittlere Spaltbreite beträgt bevorzugt von 5 bis 900 µm, besonders bevorzugt von 10 bis 600 µm. Sowohl die Eindringtiefe als auch die Spaltbreite können mit der Software Skandium (Olympus, Version 5.2) aus entsprechenden REM-Aufnahmen der Randverstärkung bestimmt werden.

In einer bevorzugten Ausführungsform der vorliegenden Erfindung ist das auf der Membran angeordnete poröse Randgebilde aus einem Polymer, ausgewählt aus der Gruppe, bestehend aus Polyethersulfon, Polyamid und Polyolefin, oder aus Gemischen hiervon aufgebaut. Bei den Polyolefinen kann es sich beispielsweise um Polyethylen (PE) oder Polypropylen (PP) handeln, ohne jedoch hierauf beschränkt zu sein. Vorzugsweise ist das poröse Randgebilde aus Polypropylen oder Polyethersulfon aufgebaut.

Grundsätzlich gilt, dass das Polymer bzw. Polymergemisch, aus welchem das poröse Randgebilde aufgebaut ist, sich bei Wärmezufuhr erweichen lassen sollte. Geeigneterweise besitzt es also einen Schmelzpunkt bzw. Schmelzbereich, welcher signifikant unterhalb der jeweiligen Zersetzungstemperatur liegt. Beispielsweise weist syndiotaktisches Polypropylen (PP-st) mit einem Kristallisationsgrad von 30 bis 40% eine Schmelztemperatur von etwa 130 °C auf, während isotaktisches Polypropylen (PP-it) mit einem Kristallisationsgrad von 70 bis 80% eine Schmelztemperatur von etwa 170 °C aufweist. Erst bei Temperaturen oberhalb von 330 °C kommt es zur Zersetzung von Polypropylen.

Die vorstehenden Ausführungen in Bezug auf das Schmelzverhalten des porösen Randgebildes gelten in gleicher Weise auch für das mindestens eine Verankerungselement des erfindungsgemäßen Filtermoduls.

In einer bevorzugten Ausführungsform der vorliegenden Erfindung ist das mindestens eine Verankerungselement aus einem Polyolefin, wie etwa Polyethylen oder Polypropylen, aufgebaut, wobei Polypropylen besonders bevorzugt ist.

Grundsätzlich ist es angeraten, dass die Membran und das poröse Randgebilde ähnliche Materialeigenschaften, beispielsweise hinsichtlich ihrer Hydrophilie bzw. Hydrophobizität, aufweisen. Dies hat den Vorteil, dass bei Vorliegen eines Überstandes nach dem Einbetten in das Verankerungselement, d.h. bei Vorliegen eines aus der Endkappe herausragenden Restverbundes aus Membran und porösem Randgebilde, möglichst ähnliche Materialien aufeinander liegen, wodurch eine verbesserte Filtrationsleistung erzielt werden kann. So ist es beispielsweise für die Flüssigfiltration vorteilhaft, dass sowohl die Membran als auch das poröse Randgebilde aus hydrophilen Materialien gebildet sind. Auch sind im Falle ähnlicher Materialeigenschaften von Membran und porösem Randgebilde die Filtermodule, was die Dichtigkeit der Systeme anbelangt, besser prüfbar.

In einer bevorzugten Ausführungsform der vorliegenden Erfindung ist die Membran aus (expandiertem) Polytetrafluorethylen aufgebaut, während das auf der Membran angeordnete poröse Randgebilde wie auch das mindestens eine Verankerungselement jeweils aus Polypropylen aufgebaut sind. In einer weiteren bevorzugten Ausführungsform der vorliegenden Erfindung, welche insbesondere als Flüssigkeitsfilter in der Virusfiltration geeignet ist, sind sowohl die Membran als auch das poröse Randgebilde jeweils aus Polyethersulfon aufgebaut, während das mindestens eine Verankerungselement aus Polypropylen aufgebaut ist.

Was die geometrische Form des mindestens einen Verankerungselements anbelangt, unterliegt das erfindungsgemäße Filtermodul keiner besonderen Einschränkung. Der Fachmann wird je nach Verwendungszweck eine geeignete geometrische Form für das mindestens eine Verankerungselement auswählen, wobei diese naturgemäß auf die geometrische Form der Membran mit dem darauf angeordneten porösen Randgebilde abgestimmt ist. Bei mehr als einem Verankerungselement kann jedes Verankerungselement je nach vorgesehenem Zweck eine individuelle geometrische Form aufweisen.

Wie vorstehend erwähnt, ist die Membran mit dem darauf angeordneten porösen Randgebilde in das mindestens eine Verankerungselement eingebettet. Der Randbereich der Membran, auf welchem das poröse Randgebilde angeordnet ist, taucht also in das mindestens eine Verankerungselement ein. Zu diesem Zweck wird das mindestens eine Verankerungselement zuvor durch Wärmezufuhr erweicht. Gemäß der vorliegenden Erfindung muss der Randbereich der Membran, auf welchem das poröse Randgebilde angeordnet ist, nicht vollständig in das mindestens eine Verankerungselement eingebettet sein. Ein (Groß-)Teil des porösen Randgebildes kann aus Stabilitätsgründen auch außerhalb des jeweiligen Verankerungselements in Form eines Überstandes verbleiben. Beispielsweise kann der Überstand, gemessen von der Einbettungsseite aus hin zur nicht-eingebetteten Filtermembran, mindestens ein 1 mm, bevorzugt mindestens 3 mm und besonders bevorzugt mindestens 5 mm betragen, wobei die Obergrenze des Überstandes 10 mm betragen kann, ohne jedoch hierauf beschränkt zu sein.

Die Tiefe der Einbettung, d.h. die Eintauchtiefe der Membran mit dem darauf angeordneten porösen Randgebilde in das mindestens eine Verankerungselement ist naturgemäß dergestalt, dass eine stabile Einbettung der Membran erreicht wird. Ansonsten unterliegt die Tiefe der Einbettung keiner besonderen Einschränkung. In einer Ausführungsform der vorliegenden Erfindung beträgt die Eintauchtiefe 0,5 bis 2,5 mm, bevorzugt 0,8 bis 2,2 mm und besonders bevorzugt 1,0 bis 2,0 mm. Ein gegebenenfalls vorhandener Überstand des porösen Randgebildes, wie vorstehend beschrieben, ist hierbei nicht berücksichtigt.

Ist das poröse Randgebilde nicht nur auf einem einzigen Randbereich der Membran angeordnet, so kann jeder Randbereich der Membran, auf welchem das poröse Randgebilde angeordnet ist, in ein entsprechendes Verankerungselement eingebettet sein. Beispielsweise kann, wie vorstehend erwähnt, das poröse Randgebilde auf zwei gegenüberliegenden Randbereichen einer rechteckigen bzw. einer darauf basierenden röhren- oder prismenförmigen Membran angeordnet sein, wobei jeder Randbereich mit dem darauf angeordneten porösen Randgebilde in ein entsprechendes Verankerungselement eingebettet ist. Das erfindungsgemäße Filtermodul umfasst in diesem Fall dann zwei Verankerungselemente. Insbesondere ist es im Fall einer röhren- oder prismenförmigen Membran bevorzugt, dass beide Randbereiche der Membran mit dem darauf angeordneten porösen Randgebilde in entsprechende Verankerungselemente eingebettet sind.

Gemäß einer weiteren bevorzugten Ausführungsform ist die Membran mit dem darauf angeordneten porösen Randgebilde plissiert. Dabei nehmen sowohl die Membran als auch das auf der Membran angeordnete poröse Randgebilde die durch die Plissierung hervorgerufene Faltenstruktur an. Liegt das poröse Randgebilde beispielsweise in Form eines Streifens vor, erfolgt die Plissierung senkrecht zur längsten Ausdehnung des Streifens. Weist die Membran mit dem darauf angeordneten porösen Randgebilde eine Röhren- oder Prismenform auf, so sind die durch die Plissierung hervorgerufenen Falten parallel zur Hauptachse der Röhre oder des Prismas ausgerichtet. Definitionsgemäß soll im Rahmen der vorliegenden Erfindung eine röhrenförmige Membran auch im Falle einer Plissierung noch als röhrenförmig gelten, selbst wenn sie streng genommen keinen kreisförmigen Querschnitt mehr aufweist.

Ist die Membran mit dem darauf angeordneten porösen Randgebilde plissiert, lässt sich im erfindungsgemäßen Filtermodul die Filteroberfläche bei gleichbleibender Größe des Filtermoduls erhöhen. Auch kann durch die Plissierung die mechanische Stabilität des Filtermoduls verbessert werden. Dies ist insbesondere dann von Vorteil, wenn die Membran aus einem vergleichsweise elastischen Material besteht, was beispielsweise auf (expandiertes) Polytetrafluorethylen zutrifft.

Gemäß einer weiteren Ausführungsform der vorliegenden Erfindung umfasst das Filtermodul neben der Membran mit dem darauf angeordneten porösen Randgebilde und neben dem mindestens einen Verankerungselement eine Rückstausicherung und einen Kern. Je nach Verwendungszweck wählt der Fachmann eine geeignete Rückstausicherung sowie einen geeigneten Kern für das Filtermodul aus.

Ferner kann das erfindungsgemäße Filtermodul noch ein bzw. mehrere Drainage- bzw. Stützvlies(e) umfassen. Typischerweise werden zwei Vliese bereitgestellt, wobei diese auf beiden Seiten der randverstärkten Filtermembran angeordnet sind, d.h. welche die Membran mit dem darauf angeordneten porösen Randgebilde umschlie-ßen und so das eigentliche Trennmedium bilden. Wie die randverstärkte Filtermembran kann auch ein gegebenenfalls vorhandenes Drainagevlies plissiert sein.

Darüber hinaus ist das erfindungsgemäße Filtermodul nicht auf eine einzige Filtermembran beschränkt. So kann das Filtermodul gemäß der vorliegenden Erfindung neben der randverstärkten Filtermembran noch mindestens eine weitere Filtermembran, welche ebenfalls randverstärkt sein kann, umfassen.

Eine typische Komponentenabfolge eines erfindungsgemäßen Filtermoduls umfasst beispielsweise - von außen nach innen - Rückstausicherung, Drainagevlies, randverstärkte Filtermembran, Drainagevlies sowie Kern, wobei zwischen den beiden Drainagevliesen noch mindestens eine weitere gegebenenfalls randverstärkte Filtermembran angeordnet sein kann. Die vorstehend genannten Komponenten sind hierbei allesamt in das mindestens eine Verankerungselement eingebettet.

In einem weiteren Aspekt betrifft die vorliegende Erfindung ein Verfahren zur Herstellung des erfindungsgemäßen Filtermoduls, wobei das Verfahren die folgenden Schritte (a) bis (h) umfasst:
(a) Bereitstellen einer Membran und eines porösen Randgebildes,
(b) Anordnen des porösen Randgebildes auf mindestens einem Randbereich auf der Membran,
(c) Verbinden der Membran mit dem darauf angeordneten porösen Randgebilde,
(d) gegebenenfalls Plissieren der Membran mit dem darauf angeordneten porösen Randgebilde,
(e) gegebenenfalls Verschweißen der Membran mit dem darauf angeordneten porösen Randgebilde entlang der Seiten, welche nicht durchgängig randverstärkt sind,
(f) Bereitstellen mindestens eines Verankerungselements,
(g) Erweichen eines Teilbereichs des mindestens einen Verankerungselements und
(h) Einbetten der Membran mit dem darauf angeordneten porösen Randgebilde in den erweichten Teilbereich des mindestens einen Verankerungselements.

Die vorstehenden Ausführungen in Bezug auf das erfindungsgemäße Filtermodul, einschließlich der Membran, des porösen Randgebildes und des mindestens einen Verankerungselements, gelten analog für das erfindungsgemäße Verfahren zur Herstellung des Filtermoduls.

Nachstehend werden die Schritte (a) bis (h) des erfindungsgemäßen Verfahrens zur Herstellung des Filtermoduls näher erläutert.

In Schritt (a) des erfindungsgemäßen Verfahrens werden eine Membran und ein poröses Randgebilde bereitgestellt. Für die Membran sowie für das poröse Randgebilde gelten alle Definitionen und Einschränkungen, wie sie vorstehend im Zusammenhang mit dem erfindungsgemäßen Filtermodul getroffen worden sind, insbesondere in Bezug auf die geometrische Form, die Porengröße sowie die Materialien, aus denen die Membran sowie das poröse Randgebilde aufgebaut sind. Soll in Schritt (e) die Membran mit dem darauf angeordneten porösen Randgebilde verschweißt werden, wird in Schritt (a) des erfindungsgemäßen Verfahrens geeigneterweise eine Membran mit rechteckiger Form bereitgestellt.

In Schritt (b) des erfindungsgemäßen Verfahrens wird das poröse Randgebilde auf mindestens einem Randbereich auf der Membran angeordnet, d.h. mindestens ein Randbereich der Membran wird durch das poröse Randgebilde bedeckt, wobei das poröse Randgebilde vorzugsweise mit dem Membranende bündig abschließt. Wie im Zusammenhang mit dem erfindungsgemäßen Filtermodul erwähnt, kann die Anordnung des porösen Randgebildes auf mehreren Randbereichen der Membran erfolgen, beispielsweise falls in Schritt (h), wie nachstehend erläutert, eine Einbettung in zwei Verankerungselemente erfolgen soll.

In Schritt (c) des erfindungsgemäßen Verfahrens wird die Membran mit dem darauf angeordneten porösen Randgebilde verbunden, was mittels Wärme- und Druckbeaufschlagung geschehen kann. Beispielsweise wird hierzu die Membran zusammen mit dem darauf angeordneten porösen Randgebilde in ein Heißsiegelungsgerät geführt und heißgesiegelt, wobei es sich hierbei um eine Randsiegelung handelt. Typischerweise erfolgt der Siegelungsprozess bei erhöhtem Druck, wobei die Membran mit dem darauf angeordneten porösen Randgebilde zunächst eine Heizpresse und anschließend eine Kühlpresse durchläuft. In der Heizpresse erfolgt unter Druck ein Zusammenpressen der Membran und des auf ihr angeordneten porösen Randgebildes bei erhöhter Temperatur, wodurch sich ein fester Verbund ausbildet, welcher im Anschluss daran in einer Kühlpresse wiederum unter Druck auf Umgebungstemperatur abgekühlt wird. Vorteilhafterweise wird bei der Siegelung keinerlei Haftmittel benötigt, da die Poren im Oberflächenbereich der Membran während des Siegelungsvorgangs mit dem erweichten Material des porösen Randgebildes teilweise gefüllt werden, was zur Stabilität des so erhaltenen Verbunds beiträgt. Typische Prozessbedingungen des Siegelungsvorgangs sind eine Temperatur von 120 bis 300 °C, ein Druck von 1 bis 5 bar sowie eine Geschwindigkeit von beispielsweise 23 × 30 mm/min, ohne jedoch hierauf beschränkt zu sein.

In einer bevorzugten Ausführungsform des erfindungsgemäßen Verfahrens wird in Schritt (c) das auf der Membran angeordnete poröse Randgebilde während des Verbindens mit einer Strukturierung bzw. Oberflächenrauheit versehen. Ist die Membran aus PTFE aufgebaut, welches ein vergleichsweise elastisches Material darstellt, und ist das poröse Randgebilde aus PP aufgebaut, so ist es bevorzugt, dass die Membran und das auf ihr angeordnete poröse Randgebilde bereits während des Heißsiegelns von einem oder mehreren Stützvliesen, beispielsweise aus Polyphenylensulfid (PPS), umgeben sind. Dies führt zum einen zu einer erhöhten Stabilität, zum anderen wird durch das Vlies eine Strukturierung bzw. Oberflächenrauheit erzeugt, da sich die Struktur des Vlieses in die Randsiegelung presst. Sind sowohl die Membran als auch das poröse Randgebilde aus PES aufgebaut, kann auch ohne Vlies heißgesiegelt werden, da PES aufgrund seiner geringeren Elastizität formstabiler ist. Um hier dennoch eine Strukturierung bzw. Oberflächenrauheit zu erzeugen, können die Membran und das auf ihr angeordnete poröse Randgebilde während des Heißsiegelns über strukturierte Förderbänder, beispielsweise über solche aus PTFE, geführt werden. Die strukturierten Förderbänder des Heißsiegelungsgeräts dienen dabei nicht nur dem Transport der Membran mit dem darauf angeordneten porösen Randgebilde durch das Heißsiegelungsgerät, sondern auch der Erzeugung eines Faserabdrucks. Die strukturierten Förderbänder, welche um die Heizbacke des Heißsiegelungsgeräts rundherum gelegt sind, "drücken" sich beim Heißsiegeln gewissermaßen in das Material ein und erzeugen auf diese Weise die Strukturierung bzw. Oberflächenrauheit der Randverstärkung. Grundsätzlich ist die Erzeugung der Strukturierung bzw. Oberflächenrauheit nicht auf die beiden vorstehend beschriebenen Ausführungsformen beschränkt. So lässt sich insbesondere im erstgenannten Fall (Membran: PTFE; poröses Randgebilde: PP) eine Strukturierung alternativ auch durch die strukturierten Förderbänder des Heißsiegelungsgeräts erreichen, wobei in diesem Fall das Heißsiegeln gänzlich ohne Vlies erfolgt. Je nach Beschaffenheit der Membran und des porösen Randgebildes wählt der Fachmann ein geeignetes Verfahren zur Erzeugung der Strukturierung bzw. Oberflächenrauheit in Schritt (c) aus, wobei hierbei auch die Beschaffenheit, insbesondere das Schmelzverhalten, gegebenenfalls vorhandener Vliese zu berücksichtigen ist.

In Schritt (d) des erfindungsgemäßen Verfahrens wird die Membran mit dem darauf angeordneten porösen Randgebilde, welche zuvor zu einem Verbund verarbeitet worden sind, gegebenenfalls plissiert. Geeignete Plissierverfahren sind dem Fachmann bekannt. Vorzugsweise wird bei einer Temperatur im Bereich von 50 bis 200 °C plissiert. Wie bereits im Zusammenhang mit dem erfindungsgemäßen Filtermodul erwähnt, erfolgt die Plissierung derart, dass sowohl die Membran als auch das auf ihrem Randbereich angeordnete poröse Randgebilde die durch die Plissierung hervorgerufene Faltenstruktur annehmen. Gegebenenfalls wird in Schritt (d) ein Drainagevlies, falls vorhanden, mitplissiert.

In Schritt (e) des erfindungsgemäßen Verfahrens wird die Membran mit dem darauf angeordneten porösen Randgebilde gegebenenfalls verschweißt, wobei das Verschweißen entlang der Seiten erfolgt, welche nicht durchgängig randverstärkt sind. Hierdurch nimmt die Membran mit dem darauf angeordneten porösen Randgebilde beispielsweise eine Röhrenform an, wobei einer der beiden Randbereiche bzw. beide Randbereiche auf der Röhrenaußenseite entlang des Röhrenumfangs mit dem porösen Randgebilde bedeckt ist/sind. Geeignete Schweißverfahren sind dem Fachmann bekannt. Insbesondere ist hier das Ultraschallschweißen zu nennen.

In Schritt (f) des erfindungsgemäßen Verfahrens wird mindestens ein Verankerungselement bereitgestellt, welches dem Abdichten der Membran mit dem darauf angeordneten porösen Randgebilde dient. Auch für das mindestens eine Verankerungselement gelten alle Definitionen und Einschränkungen, wie sie vorstehend im Zusammenhang mit dem erfindungsgemäßen Filtermodul getroffen worden sind, insbesondere in Bezug auf die geometrische Form sowie die Materialien, aus denen das mindestens eine Verankerungselement aufgebaut ist. Die geometrische Form des mindestens einen Verankerungselements ist dabei notwendigerweise auf die geometrische Form der Membran mit dem darauf angeordneten porösen Randgebilde abgestimmt. Durch geeignete Wahl der Materialien lässt sich das mindestens eine Verankerungselement durch Wärmezufuhr verformen, sodass die Membran mit dem darauf angeordneten porösen Randgebilde hierin eingebettet werden kann. Wie bereits erwähnt, ist das mindestens eine Verankerungselement besonders bevorzugt aus Polypropylen aufgebaut.

Falls erforderlich, werden neben dem mindestens einen Verankerungselement noch eine Rückstausicherung, ein Kern, mindestens eine weitere Filtermembran, welche gegebenenfalls randverstärkt ist, und ein Drainagevlies, typischerweise in doppelter Ausführung - sofern noch nicht in Schritt (c) bzw. (d) geschehen - bereitgestellt.

Nimmt die Membran mit dem darauf angeordneten porösen Randgebilde beispielsweise eine Röhrenform an, wie sie durch das Verschweißen in Schritt (e) gegebenenfalls erhalten worden ist, werden die Rückstausicherung und der Kern vor der Einbettung der Membran mit dem darauf angeordneten porösen Randgebilde in das mindestens eine Verankerungselement um die röhrenförmige Membran an- bzw. in die röhrenförmige Membran eingebracht. In analoger Weise gilt dies auch für das Drainagevlies sowie für eine gegebenenfalls vorhandene weitere Filtermembran.

In Schritt (g) des erfindungsgemäßen Verfahrens wird ein Teilbereich des mindestens einen Verankerungselements erweicht. Dies geschieht durch eine zielgerichtete Wärmezufuhr an jenen Stellen, wo die Membran mit dem darauf angeordneten porösen Randgebilde sowie gegebenenfalls vorhandene weitere Komponenten zur Einbettung eintauchen sollen. Geeignete Vorrichtungen, welche eine zielgerichtete Wärmezufuhr erlauben, sind im Stand der Technik beschrieben, wobei der Fachmann routinemäßig die Wärmezufuhr an das Material anpasst, aus welchem das mindestens eine Verankerungselement aufgebaut ist.

In Schritt (h) des erfindungsgemäßen Verfahrens wird die Membran mit dem darauf angeordneten porösen Randgebilde zusammen mit gegebenenfalls vorhandenen weiteren Komponenten in den erweichten Teilbereich des mindestens einen Verankerungselements eingebettet. Das poröse Randgebilde und der entsprechend bedeckte Randbereich der Membran müssen hierbei nicht notwendigerweise vollständig in das jeweilige Verankerungselement eintauchen. Ist das poröse Randgebilde nicht nur auf einem einzigen Randbereich der Membran angeordnet, kann in Schritt (h) eine Einbettung in mehr als ein Verankerungselement erfolgen. Handelt es sich beispielsweise um eine röhrenförmige Membran, wie sie durch das Verschweißen in Schritt (e) gegebenenfalls erhalten worden ist, können beide Randbereiche der Membran mit dem darauf angeordneten porösen Randgebilde in entsprechende Verankerungselemente eingebettet werden. Nach dem Einbetten der Membran mit dem darauf angeordneten porösen Randgebilde in das mindestens eine Verankerungselement wird das so erhaltene Filtermodul schließlich noch auf Umgebungstemperatur abgekühlt.

Vor der eigentlichen Verwendung wird das mit dem erfindungsgemäßen Verfahren hergestellte Filtermodul in einem Metall- oder Kunststoffgehäuse ("Filtercapsule") verbaut, wodurch das Filtermodul über einen definierten Ein- und Ausgang verfügt. Falls erforderlich, kann abschließend das so verbaute Filtermodul noch mittels γ-Strahlung sterilisiert werden. Typische Strahlendosen liegen im Bereich von 10 bis 200 kGy. Alternativ kann auch eine Autoklavierung oder Inline-Bedampfung bei 120 bis 140 °C für 20 bis 30 min erfolgen. Hierdurch wird sichergestellt, dass das Filtermodul frei von Keimen ist, was für eine Vielzahl von Anwendungen essentiell ist.

Aufgrund der Randverstärkung der Membran mit dem porösen Randgebilde kommt es bei der Einbettung in das mindestens eine Verankerungselement zu einer fluiddichten Verbindung der vorstehenden Komponenten, d.h. das Auftreten von Undichtigkeiten im Randbereich lässt sich hierdurch vollkommen unterbinden, wodurch das Filtermodul auch über einen längeren Benutzungszeitraum eine ausreichende Sterilität aufweist. Hierdurch eignet sich das erfindungsgemäße Filtermodul in besonderer Weise für die Steril- und Virenfiltration von fluiden Medien, insbesondere von gasförmigen Medien, vor allem von Luft, wie auch von flüssigen Medien.

In einem anderen Aspekt betrifft die vorliegende Erfindung die Verwendung des erfindungsgemäßen Filtermoduls zur Steril- oder Virenfiltration eines fluiden Mediums, wobei es sich bei dem fluiden Medium bevorzugt um ein gasförmiges Medium, besonders bevorzugt um Luft, oder um ein flüssiges Medium handelt.

Die Figuren zeigen:

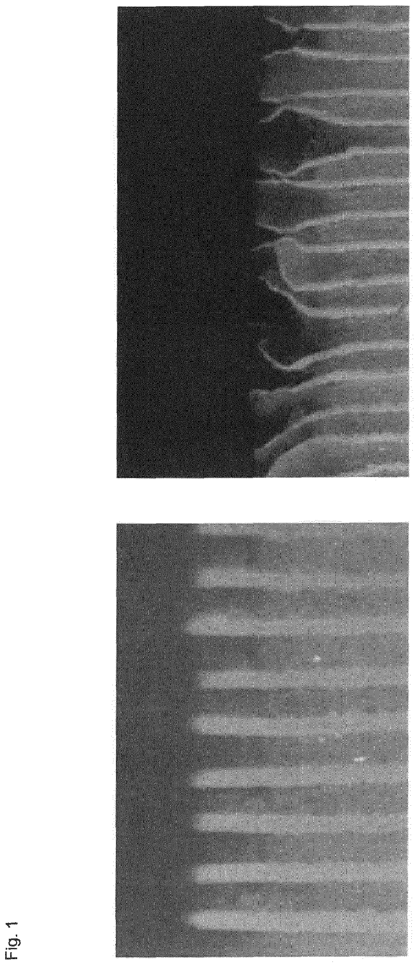

Figur 1 zeigt Rasterelektronenmikroskopie-Aufnahmen eines Filtermoduls mit randverstärkter Membran gemäß der vorliegenden Erfindung (links) sowie eines Filtermoduls ohne randverstärkte Membran als Vergleichsbeispiel (rechts). In beiden Fällen sind die Membranen plissiert. Wie Fig. 1 entnommen werden kann, ist bei Anwesenheit der Randverstärkung der Randbereich der Membran gleichmäßig mit dem Verankerungselement verzahnt, während bei Abwesenheit der Randverstärkung der Randbereich der Membran zu einem Ab- bzw. Umknicken neigt. Dies zeigt sich insbesondere in den Falten der Plissierung, welche gewissermaßen umkippen.

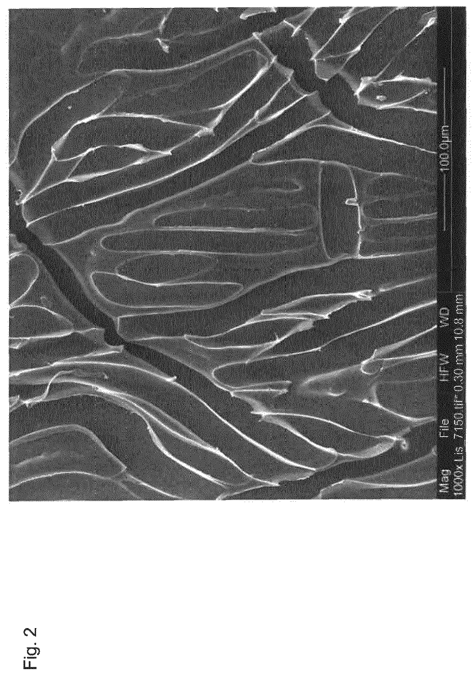

Figur 2 zeigt die Strukturierung bzw. Oberflächenrauheit beim Blick von oben am Beispiel eines erfindungsgemäßen Filtermoduls mit PTFE-Membran und darauf angeordnetem PP-Randstreifen. Wie Fig. 2 entnommen werden kann, hat sich die Struktur des Vlieses in den Randstreifen eingepresst. Anhand der REM-Aufnahme aus Fig. 2 kann die Spaltbreite der Strukturierung bzw. Oberflächenrauheit bestimmt werden.

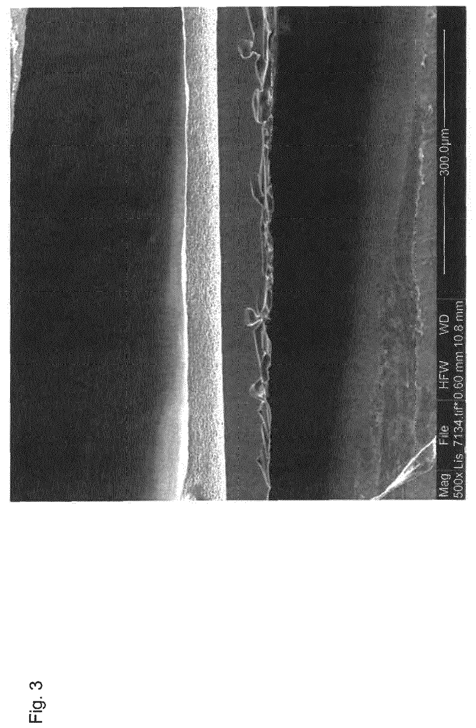

Figur 3 zeigt die durch das Vlies bedingte Strukturierung bzw. Oberflächenrauheit des erfindungsgemäßen Filtermoduls aus Fig. 2 beim Blick von der Seite. Anhand der REM-Aufnahme aus Fig. 3 kann die Eindringtiefe der Strukturierung bzw. Oberflächenrauheit bestimmt werden.

### Beispiele

Die folgenden Beispiele dienen der weiteren Erläuterung der vorliegenden Erfindung, ohne jedoch hierauf beschränkt zu sein.

### Beispiel A

Der Einfluss der Randverstärkung auf die Filtersterilität wurde anhand der bakteriellen Rückhaltung evaluiert. Hierzu wurden ein Filtermodul mit randverstärkter Membran gemäß der vorliegenden Erfindung sowie ein Filtermodul ohne randverstärkte Membran als Vergleichsbeispiel untersucht. Die Membran war in beiden Fällen aus expandiertem Polytetrafluorethylen aufgebaut. Die Randverstärkung des erfindungsgemäßen Filtermoduls, welche auf der Anströmseite aufgesiegelt wurde, war aus Polypropylen aufgebaut, ebenso die in beiden Fällen verwendeten Endkappen. Drainagevliese aus Polyphenylensulfid dienten in beiden Fällen zur Stabilisierung.

Tabelle A-1 zeigt die Sterilitätsergebnisse aus einem BC ("Bacteria Challenge")-Test für das Filtermodul ohne randverstärkte Membran (Beispiel-Nr. 1 bis 10). Der BC-Test erfolgte nach ASTM 838-05. Bei dem Filtermodul ohne randverstärkte Membran handelte es sich durchweg um 10 Zoll-Filterkerzen, wobei die ePTFE-Membran (Länge: 241 mm, Dicke: 90 ± 10 µm) eine Porengröße von 0,2 µm aufwies. Getestet wurden insgesamt jeweils fünf Kerzen aus zwei unterschiedlichen Chargen. Wie Tabelle A-1 entnommen werden kann, zeigt das Filtermodul ohne Randverstärkung bereits im unbehandelten, d.h. nicht-bedampften Zustand eine vergleichsweise hohe Keimzahl. Die Ursache hierfür liegt in der unzureichenden Verbindung der Membran mit der Endkappe (s. auch REM-Aufnahme in Fig. 1, rechts), welche letztlich zu einer mangelnden Sterilität des Filtermoduls führt.

Tabellen A-2 bis A-4 zeigen die Sterilitätsergebnisse für das Filtermodul mit randverstärkter Membran (Beispiel-Nr. 11 bis 36). Der BC-Test erfolgte auch hier nach ASTM 838-05. Auch bei dem Filtermodul mit randverstärkter Membran handelte es sich durchweg um 10 Zoll-Filterkerzen, wobei die ePTFE-Membran (Länge: 241 mm, Dicke: 90 ± 10 µm) eine Porengröße von 0,2 µm aufwies. Als poröses Randgebilde wurde eine PP-Membran (Dicke: 180 ± 20 µm) in Streifenform mit einer Porengröße von ebenfalls 0,2 µm und einem Randbereich/einer Breite von 8 mm verwendet, d.h. etwa 3,3% der Gesamtfläche der Filtermembran waren durch ein solches poröses Randgebilde bedeckt. Heißgesiegelt wurde bei einer Temperatur von 170 °C sowie bei einem Druck von 3 bar. Das Drainagevlies aus PPS wurde bereits während des Siegelungsvorgangs mitgeführt. Durch den Faserabdruck des Vlieses wurde in dem porösen Randgebilde eine Strukturierung bzw. Oberflächenrauheit erzeugt. Die mittlere Spaltbreite betrug 16 µm, während die mittlere Eindringtiefe 11 µm betrug, was sich rasterelektronenmikroskopisch ermitteln ließ (s. auch REM-Aufnahmen in Fig. 2 und in Fig. 3). Die Eintauchtiefe der Randsiegelung in die Endkappe betrug etwa 1,5 bis 2 mm, d.h. ein Überstand von 6 bis 6,5 mm ragte aus der Endkappe heraus. Getestet wurden zehn Kerzen aus einer Charge sowie vier bzw. zwölf Kerzen aus zwei weiteren Chargen. Wie Tabellen A-2 bis A-4 entnommen werden kann, ist selbst bei einer bis zu 150-fachen Bedampfung des Filtermoduls mit Randverstärkung keine signifikante Keimzahl messbar (je Zyklus 134 °C und 30 min, gefolgt von Abkühlung mit Wasser auf etwa 15 °C). Der Grund hierfür liegt in der verbesserten Verbindung der randverstärkten Membran mit der Endkappe (s. auch REM-Aufnahme in Fig. 1, links), wodurch insbesondere Undichtigkeiten im Randbereich vermieden werden können, welche sich ansonsten nachteilig auf die Sterilität des Filtermoduls auswirken würden.

### Beispiel B

Bei einem weiteren System wurde anhand der Bakteriophagenrückhaltung der Einfluss der Randverstärkung auf die Filtersterilität evaluiert. Hierzu wurden ein Filtermodul mit randverstärkter Membran gemäß der vorliegenden Erfindung sowie ein Filtermodul ohne randverstärkte Membran als Vergleichsbeispiel untersucht. Auch hier handelte es sich durchweg um 10 Zoll-Filterkerzen. Die Membran war in beiden Fällen aus Polyethersulfon aufgebaut und wies durch den Zusatz von Polyvinylpyrrolidon hydrophile Eigenschaften auf, wobei jeweils zwei Lagen einer identischen Virenmembran als Vor- und Hauptfilter verbaut waren. Die Porengröße der Filtermembranen betrug 0,02 µm. Während im erfindungsgemäßen Beispiel der Vorfilter randverstärkt war, wies der Vorfilter im Vergleichsbeispiel keine Randverstärkung auf. Die Randverstärkung, d.h. das poröse Randgebilde, des erfindungsgemäßen Filtermoduls, welche auf dem Vorfilter auf der Anströmseite aufgesiegelt wurde, war ebenfalls aus Polyethersulfon aufgebaut und wies eine Porengröße von 0,65 µm auf. Die Dicke der Vor- und Hauptfiltermembran betrug jeweils 150 ± 10 µm, während die Dicke des Randstreifens 160 ± 20 µm betrug. Heißgesiegelt wurde bei einer Temperatur von 270 °C sowie bei einem Druck von 4 bar. Die Strukturierung bzw. Oberflächenrauheit wurde durch Abdruck des strukturierten PTFE-Förderbandes des Heißsiegelungsgeräts erzeugt. Die Faserdicke des strukturierten Föderbandes lag im Bereich von 400 µm, was sich anhand von Mikroskopaufnahmen abmessen ließ. Zudem ergab sich eine Eindringtiefe von etwa 15 bis 20 µm. Der Randbereich/die Breite sowie die Eintauchtiefe in die Endkappe waren identisch zu den Werten aus Beispiel A. Sowohl im erfindungsgemäßen Beispiel als auch im Vergleichsbeispiel waren die Endkappen aus Polypropylen aufgebaut. Zudem wurden in beiden Fällen Drainagevliese aus PP verwendet.

Tabelle B zeigt die Sterilitätsergebnisse für das Filtermodul ohne randverstärkte Membran (Beispiel-Nr. I) sowie für das Filtermodul mit randverstärkter Membran (Beispiel-Nr. II bis IV). Im Fall des Filtermoduls ohne Randverstärkung wurde ein LRV ("Log10 Reduction Value") von 4,3 ermittelt. Dieser Wert entspricht im Gegensatz zu einer vollständigen Rückhaltung lediglich einer Reduktion der ursprünglichen Bakteriophagenkonzentration, d.h. es konnten noch Bakteriophagen im Filtrat nachgewiesen werden. Im Fall des erfindungsgemäßen Filtermoduls mit Randverstärkung wurde hingegen ein LRV von 6,0 ermittelt, was einer vollständigen Rückhaltung der Bakteriophagen gleichkommt. Im Vergleich zu dem Filtermodul ohne randverstärkte Membran stellt dies eine Verbesserung von etwa zwei log-Stufen dar.

Die Untersuchung der Bakteriophagenrückhaltung erfolgte hier anhand einer Phagenlösung aus *pseudomonas aeruginosa* Bakteriophagen PP7 mit einer Konzentration von mindestens 10⁷ PFU/mL, welche durch das zu testende Filtermodul bei einem konstanten Druck von 2 bar filtriert wurde. Bei der Filtration wurden die Fraktionen nicht in verschiedenen Behältern aufgefangen, sondern alle zusammen in einem Pool. Das so erhaltene Filtrat wurde anschließend auf Phagen hin untersucht.

Zur Analyse des Bakteriophagen PP7-Titers wurden Proben (150 µL-Aliquote) mit Bakterien (150 µL-Aliquot einer Übernachtkultur, in Nährlösung 1:100 verdünnt) etwa 10 Minuten bei Raumtemperatur inkubiert. Anschließend wurden 2,5 mL 0,8%-iges Agar zugegeben und das gesamte Volumen auf einer Petrischale mit 1,5%-igem festem Nähragar verteilt. Nach einer Inkubationszeit von 18 bis 24 Stunden bei 37 °C wurden die von den Bakteriophagen induzierten Beläge (Plaques) gezählt.

**Tabelle A-1**

| Bsp.-Nr. | Kerzen-Nr. | Chargen-Nr. | Behandlungsmethode | Testkeim | | beaufschlagte Keimzahl | | Keimbelastung [Keime/cm²] | | Keimzahl [CFU] | |
|---|---|---|---|---|---|---|---|---|---|---|---|
| 1 | 1 | 1 | unbehandelt | *Brevundimonas dim* | | 8,00×10¹⁰ | | 1,43×10⁷ | | 0 | |
| 2 | 2 | 1 | unbehandelt | *Brevundimonas dim* | | 8,00×10¹⁰ | | 1,43×10⁷ | | 352 | |
| 3 | 3 | 1 | unbehandelt | *Brevundimonas dim* | | 8,00×10¹⁰ | | 1,43×10⁷ | | 0 | |
| 4 | 4 | 1 | unbehandelt | *Brevundimonas dim* | | 8,00×10¹⁰ | | 1,43×10⁷ | | 40 | |
| 5 | 5 | 1 | unbehandelt | *Brevundimonas dim* | | 8,00×10¹⁰ | | 1,43×10⁷ | | 0 | |
| 6 | 1 | 2 | unbehandelt | *Brevundimonas dim* | | 8,00×10¹⁰ | | 1,43×10⁷ | | 0 | |
| 7 | 2 | 2 | unbehandelt | *Brevundimonas dim* | | 8,00×10¹⁰ | | 1,43×10⁷ | | nicht mehr zählbar | |
| 8 | 3 | 2 | unbehandelt | *Brevundimonas dim* | | 8,00×10¹⁰ | | 1,43×10⁷ | | 400 | |
| 9 | 4 | 2 | unbehandelt | *Brevundimonas dim* | | 8,00×10¹⁰ | | 1,43×10⁷ | | 338 | |
| 10 | 5 | 2 | unbehandelt | *Brevundimonas dim* | | 8,00×10¹⁰ | | 1,43×10⁷ | | 52 | |

| Bsp.-Nr. | Kerzen-Nr. | Chargen-Nr. | Membranfläche [m2] | Blasenpunkt [bar] vor Sterilisation nach BC-Test | | | Prüfdruck [bar] | | Diffusion [mL/min] vor Sterilisation nach BC-Test | | |
|---|---|---|---|---|---|---|---|---|---|---|---|
| 1 | 1 | 1 | 0,56 | 1,64 | 1,60 | | 0,70 | | 2,2 | | 1,5 |
| 2 | 2 | 1 | 0,56 | 1,59 | 1,55 | | 0,70 | | 2,1 | | 1,6 |
| 3 | 3 | 1 | 0,56 | 1,69 | 1,64 | | 0,70 | | 2,2 | | 1,5 |
| 4 | 4 | 1 | 0,56 | 1,65 | 1,63 | | 0,70 | | 2,4 | | 1,5 |
| 5 | 5 | 1 | 0,56 | 1,60 | 1,54 | | 0,70 | | 2,3 | | 1,8 |
| 6 | 1 | 2 | 0,56 | 1,60 | 1,55 | | 0,70 | | 11,0 | | 1,7 |
| 7 | 2 | 2 | 0,56 | 1,60 | 1,55 | | 0,70 | | 11,5 | | 2,8 |
| 8 | 3 | 2 | 0,56 | 1,60 | 1,59 | | 0,70 | | 11,1 | | 2,1 |
| 9 | 4 | 2 | 0,56 | 1,64 | 1,63 | | 0,70 | | 9,9 | | 1,6 |
| 10 | 5 | 2 | 0,56 | 1,64 | 1,63 | | 0,70 | | 10,7 | | 1,7 |

**Tabelle A-2**

| Bsp.-Nr. | Kerzen-Nr. | Chargen-Nr. | Behandlungsmethode | Testkeim | | beaufschlagte Keimzahl | | ) Keimbelastung [Keime/cm²] | | Keimzahl [CFU] |
|---|---|---|---|---|---|---|---|---|---|---|
| 11 | 1 | 1 | unbehandelt | *Brevundimonas dim* | | 8,75×10¹⁰ | | 1,46×10⁷ | | 0 |
| 12 | 2 | 1 | 150× bedampft | *Brevundimonas dim* | | 7,50×10¹⁰ | | 1,25×10⁷ | | 0 |
| 13 | 3 | 1 | unbehandelt | *Brevundimonas dim* | | 8,75×10¹⁰ | | 1,46×10⁷ | | 0 |
| 14 | 4 | 1 | 150× bedampft | *Brevundimonas dim* | | 7,50×10¹⁰ | | 1,25×10⁷ | | 0 |
| 15 | 5 | 1 | 150× bedampft | *Brevundimonas dim* | | 7,50×10¹⁰ | | 1,25×10⁷ | | 0 |
| 16 | 6 | 1 | 150× bedampft | *Brevundimonas dim* | | 7,50×10¹⁰ | | 1,25×10⁷ | | 0 |
| 17 | 7 | 1 | unbehandelt | *Brevundimonas dim* | | 8,75×10¹⁰ | | 1,46×10⁷ | | 0 |
| 18 | 8 | 1 | 150× bedampft | *Brevundimonas dim* | | 7,50×10¹⁰ | | 1,25×10⁷ | | 0 |
| 19 | 9 | 1 | unbehandelt | *Brevundimonas dim* | | 8,75×10¹⁰ | | 1,46×10⁷ | | 0 |
| 20 | 10 | 1 | unbehandelt | *Brevundimonas dim* | | 8,75×10¹⁰ | | 1,46×10⁷ | | 0 |

| Bsp.-Nr. | Kerzen-Nr. | Chargen-Nr. | Membranfläche [m2] | Blasenpunkt [bar] vor Sterilisation nach BC-Test | | | Prüfdruck [bar] | | Diffusion [mL/min] vor Sterilisation nach BC-Test | |
|---|---|---|---|---|---|---|---|---|---|---|
| 11 | 1 | 1 | 0,60 | --- | --- | | 0,70 | | --- | 2,6 |
| 12 | 2 | 1 | 0,60 | --- | 1,59 | | 0,70 | | --- | 2,9 |
| 13 | 3 | 1 | 0,60 | --- | --- | | 0,70 | | --- | 2,9 |
| 14 | 4 | 1 | 0,60 | --- | 1,58 | | 0,70 | | --- | 2,1 |
| 15 | 5 | 1 | 0,60 | --- | 1,60 | | 0,70 | | --- | 1,4 |
| 16 | 6 | 1 | 0,60 | --- | 1,59 | | 0,70 | | --- | 5,4 |
| 17 | 7 | 1 | 0,60 | --- | --- | | 0,70 | | --- | 3,3 |
| 18 | 8 | 1 | 0,60 | --- | 1,54 | | 0,70 | | --- | 2,9 |
| 19 | 9 | 1 | 0,60 | --- | --- | | 0,70 | | --- | 2,8 |
| 20 | 10 | 1 | 0,60 | --- | --- | | 0,70 | | --- | 2,6 |

**Tabelle A-3**

| Bsp.-Nr. | Kerzen-Nr. | Chargen-Nr. | Behandlungs-methode | Test-keim | | beaufschlagte Keimzahl | | Keimbelastung [Keime/cm²] | | Keimzahl [CFU] |
|---|---|---|---|---|---|---|---|---|---|---|
| 21 | 1 | 2 | 25× bedampft | *Brevundimonas dim* | | 8,00×10¹⁰ | | 1,33×10⁷ | | 0 |
| 22 | 2 | 2 | unbehandelt | *Brevundimonas dim* | | 7,75×10¹⁰ | | 1,29×10⁷ | | 0 |
| 23 | 3 | 2 | unbehandelt | *Brevundimonas dim* | | 7,75×10¹⁰ | | 1,29×10⁷ | | 0 |
| 24 | 4 | 2 | 25× bedampft | *Brevundimonas dim* | | 8,00×10¹⁰ | | 1,33×10⁷ | | 0 |
| 25 | 1 | 3 | unbehandelt | *Brevundimonas dim* | | 7,25×10¹⁰ | | 1,32×10⁷ | | 0 |
| 26 | 2 | 3 | unbehandelt | *Brevundimonas dim* | | 7,25×10¹⁰ | | 1,32×10⁷ | | 0 |
| 27 | 3 | 3 | unbehandelt | *Brevundimonas dim* | | 7,25×10¹⁰ | | 1,32×10⁷ | | 0 |
| 28 | 4 | 3 | unbehandelt | *Brevundimonas dim* | | 7,25×10¹⁰ | | 1,32×10⁷ | | 0 |
| 29 | 5 | 3 | unbehandelt | Brevundimonas dim | | 7,25×10¹⁰ | | 1,32×10⁷ | | 0 |
| 30 | 6 | 3 | unbehandelt | *Brevundimonas dim* | | 7,25×10¹⁰ | | 1,32×10⁷ | | 0 |

| Bsp.-Nr. | Kerzen-Nr. | Chargen-Nr. | Membranfläche [m2] | Blasenpunkt [bar] vor Sterilisation nach BC-Test | | | Prüfdruck [bar] | | Diffusion [mLlmin] vor Sterilisation nach BC-Test | |
|---|---|---|---|---|---|---|---|---|---|---|
| 21 | 1 | 2 | 0,60 | --- | 1,59 | | 0,70 | | --- | 3,8 |
| 22 | 2 | 2 | 0,60 | --- | 1,65 | | 0,70 | | --- | 3,5 |
| 23 | 3 | 2 | 0,60 | --- | 1,65 | | 0,70 | | --- | 2,1 |
| 24 | 4 | 2 | 0,60 | --- | 1,59 | | 0,70 | | --- | 3,0 |
| 25 | 1 | 3 | 0,55 | --- | 1,55 | | 0,70 | | --- | 1,0 |
| 26 | 2 | 3 | 0,55 | --- | 1,54 | | 0,70 | | --- | 2,0 |
| 27 | 3 | 3 | 0,55 | --- | 1,49 | | 0,70 | | --- | 1,7 |
| 28 | 4 | 3 | 0,55 | --- | 1,55 | | 0,70 | | --- | 2,6 |
| 29 | 5 | 3 | 0,55 | --- | 1,53 | | 0,70 | | --- | 1,9 |
| 30 | 6 | 3 | 0,55 | --- | 1,50 | | 0,70 | | --- | 1,9 |

**Tabelle A-4**

| Bsp.-Nr. | Kerzen-Nr. | Chargen-Nr. | Behandlungsmethode | Testkeim | | beaufschlagte Keimzahl | | Keimbelastung [Keime/cm²] | | Keimzahl [CFU] |
|---|---|---|---|---|---|---|---|---|---|---|
| 31 | 7 | 3 | unbehandelt | *Brevundimonas dim* | | 8,50×10¹⁰ | | 1,55×10⁷ | | 0 |
| 32 | 8 | 3 | unbehandelt | *Brevundimonas dim* | | 8,50×10¹⁰ | | 1,55×10⁷ | | 0 |
| 33 | 9 | 3 | unbehandelt | *Brevundimonas dim* | | 8,50×10¹⁰ | | 1,55×10⁷ | | 0 |
| 34 | 10 | 3 | unbehandelt | *Brevundimonas dim* | | 8,50×10¹⁰ | | 1,55×10⁷ | | 0 |
| 35 | 11 | 3 | unbehandelt | *Brevundimonas dim* | | 8,50×10¹⁰ | | 1,55×10⁷ | | 0 |
| 36 | 12 | 3 | unbehandelt | *Brevundimonas dim* | | 8,50×10¹⁰ | | 1,55×10⁷ | | 0 |

| Bsp.-Nr. | Kerzen-Nr. | Chargen-Nr. | Membranfläche [m2] | Blasenpunkt [bar] vor Sterilisation nach BC-Test | | | Prüfdruck [bar] | | Diffusion [mL/min] vor Sterilisation nach BC-Test | |
|---|---|---|---|---|---|---|---|---|---|---|
| 31 | 7 | 3 | 0,55 | --- | 1,54 | | 0,70 | | --- | 2,2 |
| 32 | 8 | 3 | 0,55 | --- | 1,50 | | 0,70 | | --- | 1,8 |
| 33 | 9 | 3 | 0,55 | --- | 1,55 | | 0,70 | | --- | 2,2 |
| 34 | 10 | 3 | 0,55 | --- | 1,50 | | 0,70 | | --- | 4,3 |
| 35 | 11 | 3 | 0,55 | --- | 1,54 | | 0,70 | | --- | 2,2 |
| 36 | 12 | 3 | 0,55 | --- | 1,55 | | 0,70 | | --- | 1,7 |

**Tabelle B**

| Bsp.-Nr. | Chargen-Nr. | Filterfläche [cm2] | Vorfilter | Hauptfilter | Prä-Diffusion [mL/min] | Post-Diffusion [mL/min] |
|---|---|---|---|---|---|---|
| I | 1 | 6500 | nicht gesiegelt | nicht gesiegelt | 11,0 | 9,5 |
| II | 1 | 5189 | gesiegelt | nicht gesiegelt | 11,0 | 11,4 |
| III | 2 | 5497 | gesiegelt | nicht gesiegelt | 10,6 | 11,4 |
| IV | 2 | 5497 | gesiegelt | nicht gesiegelt | 9,6 | 10,9 |

| Bsp.-Nr. | Chargen-Nr. | Fraktionen [mL] | Titer L2 [PFU/mL] | Titer Fraktionen [PFU/mL] | Plaques? | LRV |
|---|---|---|---|---|---|---|
| I | 1 | Pool | 1,8×10⁷ | 8,5×10² | Bakteriophagen im Filtrat | 4,3 |
| II | 1 | Pool | 1,8×10⁷ | 2,0×10¹ (*) | vollständiger Rückhalt | 6,0 |
| III | 2 | Pool | 1,8×10⁷ | 2,0×10¹ (*) | vollständiger Rückhalt | 6,0 |
| IV | 2 | Pool | 1,8×10⁷ | 2,0×10¹ (*) | vollständiger Rückhalt | 6,0 |

| | | | | | | |
|---|---|---|---|---|---|---|
| (*) Es wurden keine Phagen im Filtrat detektiert. Die angegebenen Werte stellen berechnete statistische Werte dar, um das Detektionslimit abschätzen zu können (s. auch "Guidance for industry - Q5A viral safety evaluation of biotechnology products derived from cell lines of human or animal origin"). | | | | | | |

Die vorliegende Erfindung erlaubt die Bereitstellung eines Filtermoduls, welches auf einer randverstärkten Membran beruht. Durch die Randverstärkung wird eine fluiddichte Verbindung der Filtermembran mit dem Verankerungselement erreicht. Insbesondere kann durch das erfindungsgemäße Herstellungsverfahren das Auftreten von Undichtigkeiten im Randbereich des Filtermoduls nahezu vollständig unterbunden werden, welche sich anderenfalls nachteilig auf die Sterilität des Filtermoduls auswirken würden. Vorteilhafterweise kann hierdurch eine ausreichende Sterilität auch über einen längeren Benutzungszeitraum gewährleistet werden, wodurch sich das erfindungsgemäße Filtermodul für all jene Anwendungen eignet, die einer Bakterien- und Virenfreiheit bedürfen. So ist das erfindungsgemäße Filtermodul hervorragend für die Steril- und Virenfiltration von fluiden Medien, insbesondere von gasförmigen Medien, vor allem von Luft, wie auch von flüssigen Medien geeignet.

## Patentansprüche

1. Filtermodul, umfassend eine Filtermembran mit einem darauf angeordneten porösen Randgebilde, welches einen Randbereich der Filtermembran derart bedeckt, dass die Filtermembran nicht vollständig bedeckt ist, und weiter umfassend mindestens ein Verankerungselement, wobei die Filtermembran mit dem darauf angeordneten porösen Randgebilde in das mindestens eine Verankerungselement eingebettet ist, die Filtermembran und das darauf angeordnete poröse Randgebilde als Verbund vorliegen und das auf der Filtermembran angeordnete poröse Randgebilde eine Porengröße von mindestens 0,1 µm aufweist.

2. Filtermodul nach Anspruch 1, wobei das auf der Filtermembran angeordnete poröse Randgebilde eine Strukturierung bzw. Oberflächenrauheit aufweist.

3. Filtermodul nach Anspruch 1 oder 2, wobei ein Teil des porösen Randgebildes außerhalb des mindestens einen Verankerungselements in Form eines Überstandes verbleibt.

4. Filtermodul nach einem der Ansprüche 1 bis 3, wobei die Filtermembran mit dem darauf angeordneten porösen Randgebilde röhrenförmig ist.

5. Filtermodul nach einem der Ansprüche 1 bis 4, wobei die Filtermembran mit dem darauf angeordneten porösen Randgebilde plissiert ist.

6. Filtermodul nach einem der Ansprüche 1 bis 5, wobei die Filtermembran aus einem Polymer, ausgewählt aus der Gruppe, bestehend aus Polytetrafluorethylen, Polyvinylidenfluorid, Polyethersulfon, Polyamid, Polyolefin, Celluloseacetat und Cellulose, oder aus Gemischen hiervon aufgebaut ist.

7. Filtermodul nach einem der Ansprüche 1 bis 6, wobei das auf der Filtermembran angeordnete poröse Randgebilde aus einem Polymer, ausgewählt aus der Gruppe, bestehend aus Polyethersulfon, Polyamid und Polyolefin, oder aus Gemischen hiervon aufgebaut ist.

8. Filtermodul nach einem der Ansprüche 1 bis 7, wobei die Filtermembran aus Polytetrafluorethylen aufgebaut ist und das poröse Randgebilde sowie das mindestens eine Verankerungselement jeweils aus Polypropylen aufgebaut sind.

9. Filtermodul nach einem der Ansprüche 1 bis 7, wobei die Filtermembran sowie das poröse Randgebilde jeweils aus Polyethersulfon aufgebaut sind und das mindestens eine Verankerungselement aus Polypropylen aufgebaut ist.

10. Filtermodul nach einem der Ansprüche 1 bis 9, wobei das poröse Randgebilde ein poröser Film oder eine Membran ist.

11. Verfahren zur Herstellung des Filtermoduls nach einem der Ansprüche 1 bis 10, umfassend die folgenden Schritte:
(a) Bereitstellen einer Filtermembran und eines porösen Randgebildes,
(b) Anordnen des porösen Randgebildes auf mindestens einem Randbereich auf der Filtermembran,
(c) Verbinden der Filtermembran mit dem darauf angeordneten porösen Randgebilde,
(d) gegebenenfalls Plissieren der Filtermembran mit dem darauf angeordneten porösen Randgebilde,
(e) gegebenenfalls Verschweißen der Filtermembran mit dem darauf angeordneten porösen Randgebilde entlang der Seiten, welche nicht durchgängig randverstärkt sind,
(f) Bereitstellen mindestens eines Verankerungselements,
(g) Erweichen eines Teilbereichs des mindestens einen Verankerungselements und
(h) Einbetten der Filtermembran mit dem darauf angeordneten porösen Randgebilde in den erweichten Teilbereich des mindestens einen Verankerungselements.

12. Verfahren nach Anspruch 11, wobei in Schritt (c) das auf der Filtermembran angeordnete poröse Randgebilde während des Verbindens mit einer Strukturierung bzw. Oberflächenrauheit versehen wird.

13. Verwendung des Filtermoduls nach einem der Ansprüche 1 bis 10 zur Steril- oder Virenfiltration eines fluiden Mediums.

14. Verwendung nach Anspruch 13, wobei das fluide Medium Luft ist.

## Claims

1. Filter module comprising a filter membrane having a porous edge structure arranged thereon which covers an edge region of the filter membrane in such a way, that the membrane is not completely covered, and further at least one anchoring element, wherein the filter membrane that has the porous edge structure arranged thereon is embedded in the at least one anchoring element, the filter membrane and the porous edge structure arranged thereon are present as a composite and the porous edge structure arranged on the membrane has a pore size of at least 0.1 µm.

2. Filter module according to claim 1, wherein the porous edge structure arranged on the membrane is textured or has a surface roughness.

3. Filter module according to claim 1 or 2, wherein part of the porous edge structure remains outside the at least one anchoring element, forming a protrusion.

4. Filter module according to anyone of claims 1 to 3, wherein the membrane having the porous edge structure arranged thereon is tubular.

5. Filter module according to anyone of claims 1 to 4, wherein the membrane having the porous edge structure arranged thereon is pleated.

6. Filter module according to anyone of claims 1 to 5, wherein the membrane is constructed from a polymer selected from the group consisting of polytetrafluoroethylene, polyvinylidene fluoride, polyethersulfone, polyamide, polyolefin, cellulose acetate, cellulose, and mixtures thereof.

7. Filter module according to anyone of claims 1 to 6, wherein the porous edge structure arranged on the membrane is constructed from a polymer selected from the group consisting of polyethersulfone, polyamide, polyolefin, and mixtures thereof.

8. Filter module according to anyone of claims 1 to 7, wherein the membrane is constructed from polytetrafluoroethylene, and wherein both the porous edge structure and the at least one anchoring element are constructed from polypropylene.

9. Filter module according to anyone of claims 1 to 7, wherein both the membrane and the porous edge structure are constructed from polyethersulfone, and wherein the at least one anchoring element is constructed from polypropylene.

10. Filter module according to anyone of claims 1 to 9, wherein the porous edge structure is a porous film or a membrane.

11. Method for producing the filter module according to anyone of claims 1 to 10, comprising the following steps:
(a) providing a membrane and a porous edge structure,
(b) arranging the porous edge structure on at least one edge region on the membrane,
(c) connecting the membrane to the porous edge structure arranged thereon,
(d) optionally pleating the membrane having the porous edge structure arranged thereon,
(e) optionally welding the membrane having the porous edge structure arranged thereon along the sides which do not have continuous edge reinforcement,
(f) providing at least one anchoring element,
(g) softening a subregion of the at least one anchoring element, and
(h) embedding the membrane having the porous edge structure arranged thereon in the softened subregion of the at least one anchoring element.

12. Method according to claim 11, wherein the porous edge structure arranged on the membrane is textured or is provided with a surface roughness during the connection operation in step (c).

13. Use of the filter module according to anyone of claims 1 to 10 for sterile-filtration and virus filtration of a fluid medium.

14. Use according to claim 13, wherein the fluid medium is air.

## Revendications

1. Module de filtration, comprenant une membrane de filtration avec une structure de bord poreuse disposée par-dessus, qui recouvre une zone de bord de la membrane de filtration de sorte que la membrane de filtration n'est pas entièrement recouverte, et comprenant en outre au moins un élément d'ancrage, dans lequel la membrane de filtration avec la structure de bord poreuse disposée par-dessus est incorporée dans le au moins un élément d'ancrage, la membrane de filtration et la structure de bord poreuse disposée par-dessus se présentent sous forme de composite et la structure de bord poreuse disposée sur la membrane de filtration présente une taille de pores d'au moins 0,1 pm.

2. Module de filtration selon la revendication 1, dans lequel la structure de bord poreuse disposée sur la membrane de filtration présente une structuration et/ou une rugosité de surface.

3. Module de filtration selon la revendication 1 ou 2, dans lequel une partie de la structure de bord poreuse subsiste en dehors du au moins un élément d'ancrage sous la forme d'une saillie.

4. Module de filtration selon l'une des revendications 1 à 3, dans lequel la membrane de filtration avec la structure de bord poreuse disposée par-dessus est de forme tubulaire.

5. Module de filtration selon l'une des revendications 1 à 4, dans lequel la membrane de filtration avec la structure de bord poreuse disposée par-dessus est plissée.

6. Module de filtration selon l'une des revendications 1 à 5, dans lequel la membrane de filtration est constituée à partir d'un polymère, choisi dans le groupe consistant en polytétrafluoroéthylène, fluorure de polyvinylidène, polyéther-sulfone, polyamide, polyoléfine, acétate de cellulose et cellulose, ou des mélanges de ceux-ci.

7. Module de filtration selon l'une des revendications 1 à 6, dans lequel la structure de bord poreuse disposée sur la membrane de filtration est constituée à partir d'un polymère choisi dans le groupe consistant en polyéther-sulfone, polyamide et polyoléfine, ou des mélanges de ceux-ci.

8. Module de filtration selon l'une des revendications 1 à 7, dans lequel la membrane de filtration est constituée à partir de polytétrafluoroéthylène et la structure de bord poreuse ainsi que le au moins un élément d'ancrage sont respectivement constitués à partir de polypropylène.

9. Module de filtration selon l'une des revendications 1 à 7, dans lequel la membrane de filtration ainsi que la structure de bord poreuse sont constituées respectivement à partir de polyéther-sulfone et le au moins un élément d'ancrage est constitué à partir de polypropylène.

10. Module de filtration selon l'une des revendications 1 à 9, dans lequel la structure de bord poreuse est un film poreux ou une membrane.

11. Procédé de fabrication du module de filtration selon l'une des revendications 1 à 10, comprenant les étapes suivantes de :
(a) mise à disposition d'une membrane de filtration et d'une structure de bord poreuse,
(b) disposition de la structure de bord poreuse sur au moins une zone de bord sur la membrane de filtration,
(c) liaison de la membrane de filtration avec la structure de bord poreuse disposée par-dessus,
(d) le cas échéant plissement de la membrane de filtration avec la structure de bord poreuse disposée par-dessus,
(e) le cas échéant soudage de la membrane de filtration avec la structure de bord poreuse disposée par-dessus le long des côtés qui ne sont pas renforcés au bord de manière continue,
(f) mise à disposition d'au moins un élément d'ancrage,
(g) ramollissement d'une zone partielle du au moins un élément d'ancrage et
(h) incorporation de la membrane de filtration avec la structure de bord poreuse disposée par-dessus dans la zone partielle ramollie du au moins un élément d'ancrage.

12. Procédé selon la revendication 11, dans lequel, à l'étape (c), la structure de bord poreuse disposée sur la membrane de filtration est dotée d'une structuration et/ou rugosité de surface pendant la liaison.

13. Utilisation du module de filtration selon l'une des revendications 1 à 10 pour la filtration stérile ou virale d'un milieu fluide.

14. Utilisation selon la revendication 13, dans laquelle le milieu fluide est l'air.
